(19) Europäisches Patentamt
European Patent Office
Office européen des brevets

(11) EP 1 567 135 B1

# (12) EUROPÄISCHE PATENTSCHRIFT

(45) Veröffentlichungstag und Bekanntmachung des Hinweises auf die Patenterteilung:
**23.07.2008 Patentblatt 2008/30**

(21) Anmeldenummer: **03785648.1**

(22) Anmeldetag: **19.11.2003**

(51) Int Cl.:
***A61K 9/00*** *(2006.01)* ***A61K 9/72*** *(2006.01)*
***A61K 31/46*** *(2006.01)*

(86) Internationale Anmeldenummer:
**PCT/EP2003/012911**

(87) Internationale Veröffentlichungsnummer:
**WO 2004/047796 (10.06.2004 Gazette 2004/24)**

## (54) TIOTROPIUMHALTIGE PULVERFORMULIERUNG FÜR DIE INHALATION

POWDER FORMULATION FOR INHALATION CONTAINING TIOTROPIUM

FORMULATION PULVERULENTE POUR INHALATION CONTENANT DU TIOTROPIUM

(84) Benannte Vertragsstaaten:
**AT BE BG CH CY CZ DE DK EE ES FI FR GB GR HU IE IT LI LU MC NL PT RO SE SI SK TR**
Benannte Erstreckungsstaaten:
**AL LT LV MK**

(30) Priorität: **28.11.2002 DE 10255387**

(43) Veröffentlichungstag der Anmeldung:
**31.08.2005 Patentblatt 2005/35**

(73) Patentinhaber: **Boehringer Ingelheim Pharma GmbH & Co. KG**
**55216 Ingelheim (DE)**

(72) Erfinder:
- **HARTIG, Mareke**
  **55218 Ingelheim (DE)**
- **TRUNK, Michael**
  **55218 Ingelheim (DE)**
- **WALZ, Michael**
  **55411 Bingen (DE)**

(56) Entgegenhaltungen:
**WO-A-00/28979** **WO-A-00/47200**
**WO-A-02/30389** **WO-A-02/30390**
**WO-A-93/11746** **WO-A-94/28958**
**WO-A-95/11666** **WO-A-96/02231**
**WO-A-02/098874** **WO-A-03/017970**
**WO-A-03/084502** **WO-A-03/084509**
**FR-M- 8 142**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 567 135 B1

## Beschreibung

Die Erfindung betrifft Tiotropium enthaltende pulverförmige Zubereitungen für die Inhalation, Verfahren zu deren Herstellung sowie deren Verwendung zur Herstellung eines Arzneimittels zur Behandlung von Atemwegserkrankungen, insbesondere zur Behandlung von COPD (chronic obstructive pulmonary disease = chronisch obstruktive Lungenerkrankung) und Asthma.

Hintergrund der Erfindung

Tiotropiumbromid ist aus der Europäischen Patentanmeldung EP 418 716 A1 bekannt und weist die folgende chemische Struktur auf:

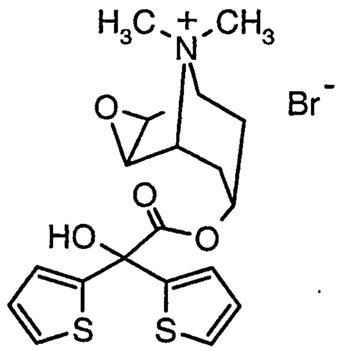

Tiotropiumbromid stellt ein hoch wirksames Anticholinergikum mit langanhaltender Wirkdauer dar, welches zur Therapie von Atemwegserkrankungen, insbesondere von COPD (chronic obstructive pulmonary disease = chronisch obstruktive Lungenerkrankung) und Asthma Verwendung finden kann. Unter Tiotropium ist das freie Ammoniumkation zu verstehen.

Bei der Behandlung vorstehender Erkrankungen bietet sich die inhalative Applikation des Wirkstoffs an. Neben der inhalativen Applikation von broncholytisch wirksamen Verbindungen in Form von Dosieraerosolen und Lösungen zur Inhalation kommt der Applikation von wirkstoffhaltigen Inhalationspulvern besondere Bedeutung zu.

Bei Wirkstoffen, die eine besonders hohe Wirksamkeit aufweisen, sind pro Einzeldosis zur Erzielung des therapeutisch erwünschten Effekts nur geringe Mengen des Wirkstoffs erforderlich. In solchen Fällen ist es notwendig, zur Herstellung des Inhalationspulvers den Wirkstoff mit geeigneten Hilfsstoffen zu verdünnen. Aufgrund des hohen Anteils an Hilfsstoff werden die Eigenschaften des Inhalationspulvers maßgeblich durch die Wahl des Hilfsstoffs beeinflußt. Bei der Wahl des Hilfsstoffs kommt dessen Korngröße eine besondere Bedeutung zu. Je feiner der Hilfsstoff desto schlechter sind in der Regel dessen Fließeigenschaften. Gute Fließeigenschaften sind allerdings Voraussetzung für eine hohe Dosiergenauigkeit bei der Abfüllung und Abteilung der einzelnen Präparatedosen, wie etwa bei der Herstellung von Kapseln (Inhaletten) zur Pulverinhalation oder der Dosierung eines Einzelhubes durch den Patienten vor der Anwendung eines Mehrdosisinhalators. Des weiteren ist die Korngröße des Hilfsstoffs von großer Bedeutung für das Entleerungsverhalten von Kapseln in einem Inhalator bei der Anwendung. Es hat sich ferner gezeigt, daß die Korngröße des Hilfsstoffs starken Einfluß auf den ausgebrachten inhalierbaren Wirkstoffanteil des Inhalationspulvers hat. Unter inhalierbarem bzw. inhalierfähigem Wirkstoffanteil werden die Teilchen des Inhalationspulvers verstanden, die beim Inhalieren mit der Atemluft tief in die Verästelungen der Lunge transportiert werden. Die hierzu erforderliche Teilchengröße liegt zwischen 1 und 10μm, vorzugsweise unter 6 μm.

Es ist Aufgabe der Erfindung, ein Tiotropium-haltiges Inhaltionspulver bereitzustellen, welches bei guter Dosiergenauigkeit (betreffend die pro Kapsel herstellerseitig abgefüllte Menge an Wirkstoff und Pulvermischung wie auch die pro Kapsel durch den Inhalationsvorgang ausgebrachte und lungengängige Wirkstoffmenge) und geringer chargenweisen Variabilität die Applikation des Wirkstoffs mit hohem inhalierfähigen Anteil erlaubt. Es ist ferner Aufgabe der vorliegenden Erfindung, ein Tiotropium-haltiges Inhaltionspulver bereitzustellen, welches ein gutes Entleerungsverhalten der Kapseln gewährleistet, sollte es z.B. mittels eines Inhalators, wie er beispielsweise in der WO 94/28958 beschrieben wird, am Patienten oder in vitro über einen Impaktor oder Impinger zur Anwendung gelangen.

Daß Tiotropium, insbesondere Tiotropiumbromid, bereits in sehr geringen Dosen eine hohe therapeutische Wirksamkeit aufweist, stellt weitere Anforderungen an ein mit hoher Dosiergenauigkeit einzusetzendes Inhalationspulver. Aufgrund der geringen, zur Erzielung des therapeutischen Effekts erforderlichen Konzentration des Wirkstoffs im Inhalationspulver, muß ein hohes Maß an Homogenität der Pulvermischung und eine geringe Schwankung im Dispergierverhalten von Pulverkapsel- zu Pulverkapselcharge gewährleistet werden. Die Homogenität der Pulvermischung wie auch gering schwankende Dispergiereigenschaften tragen entscheidend dazu bei, daß die Freisetzung des inhalierfähigen Anteils des Wirkstoffs reproduzierbar in gleichbleibend hohen Mengen und somit möglichst geringer Variabilität erfolgt.

Dementsprechend ist es ferner Aufgabe der vorliegenden Erfindung, ein Tiotropium-haltiges Inhaltionspulver bereitzustellen, welches durch eine hohes Maß an Homogenität und Gleichförmigkeit der Dispergierbarkeit gekennzeichnet ist. Ferner zielt die vorliegende Erfindung auf die Bereitstellung eines Inhalationspulvers, welches die Applikation des inhalierfähigen Wirkstoffanteils bei möglichst geringer Variabilität erlaubt.

Tiotropium-haltige Inhalationspulver, die den vorstehend genannten Aufgaben gerecht werden, sind beispielsweise aus der WO 02/30389 bekannt. Diese Inhalationspulver sind im wesentlichen dadurch gekennzeichnet, daß sie neben dem Wirkstoff Tiotropium in Form eines der von Tiotropium gebildeten pharmakologisch verträglichen Salze einen Hilfsstoff enthalten, der durch Mischung von feineren Hilfsstofffraktionen mit gröberen Hilfsstofffraktionen erhalten wird. Zur Darstellung dieser aus der WO 02/30389 bekannten Inhalationspulver sind allerdings technisch aufwendige Herstell- bzw. Mischverfahren erforderlich. Es ist daher eine weitere Aufgabe der vorliegenden Erfindung, Inhalationspulver bereitzustellen, die nicht nur die vorstehend genannten Aufgaben lösen, sondern ferner durch eine einfachere technische Darstellungsweise gewonnen werden können.

Wenn auch nicht ausschließlich, so aber doch insbesondere bei der Applikation von Inhalationspulvern mittels pulver-haltiger Kapseln, spielt das Entleerungsverhalten aus dem Pulverreservoir (das Behältnis, aus dem heraus das Wirkstoff-haltige Inhalationspulver zur inhalativen Applikation freigesetzt wird) eine bedeutsame Rolle. Wird die Pulverformulierung aus dem Pulverreservoir aufgrund eines nur geringen bzw. schlechten Entleerungsverhaltens nur in geringem Maße freigesetzt, bleiben bedeutsame Mengen des wirkstoffhaltigen Inhalationspulvers im Pulverreservoir (z.B. der Kapsel) zurück und können durch den Patienten nicht therapeutisch nutzbar gemacht werden. Dies hat zur Konsequenz, daß die Dosierung des Wirkstoffs in der Pulvermischung erhöht werden muß, damit die ausgebrachte Wirkstoffmenge zur Erzielung des therapeutisch gewünschten Effekts ausreichend hoch ist.

Vor diesem Hintergrund ist es eine weitere Aufgabe der vorliegenden Erfindung, eine Inhalationspulver bereitzustellen, welches ferner durch ein sehr gutes Entleerungsverhalten gekennzeichnet ist.

<u>Detaillierte Beschreibung der Erfindung</u>

Überraschenderweise wurde gefunden, daß die eingangs genannten Aufgaben durch die nachstehend beschriebenen, erfindungsgemäßen pulverförmigen Zubereitungen für die Inhalation (Inhalationspulver) gelöst werden.

Dementsprechend zielt die vorliegende Erfindung auf Inhalationspulver enthaltend 0,001 bis 3 % Tiotropium im Gemisch mit einem physiologisch unbedenklichen Hilfsstoff, dadurch gekennzeichnet, daß der Hilfsstoff eine mittlere Teilchengröße von 10 - 50 $\mu$m, einen 10 %-Feinanteil von 0,5 bis 6 $\mu$m sowie eine spezifischen Oberfläche von 0,1 bis 2 $m^2/g$ aufweist.

Dabei wird unter der mittleren Teilchengröße im hier verwendeten Sinne der 50 % - Wert aus der Volumenverteilung gemessen mit einem Laserdiffraktometer nach der Trockendispersionsmethode verstanden. Analog ist unter dem 10 % - Feinanteil im hier verwendeten Sinne der 10 % - Wert aus der mit einem Laserdiffraktometer gemessenen Volumenverteilung zu verstehen. Mit anderen Worten steht im Sinne der vorliegenden Erfindung der 10%-Feinanteil-Wert für die Teilchengröße, unterhalb derer 10% der Teilchenmenge liegt (bezogen auf Volumenverteilung).

Unter spezifischer Oberfläche wird im Sinne der Erfindung die massenspezifische Pulveroberfläche verstanden, berechnet aus der $N_2$-Absorptions-Isotherme, die bei dem Kochpunkt von flüssigem Stickstoff beobachtet wird (Methode von Brunauer, Emmett und Teller).

Erfindungsgemäß bevorzugt sind Inhalationspulver, die 0,01 bis 2 % Tiotropium enthalten. Besonders bevorzugte Inhalationspulver enthalten Tiotropium in einer Menge von etwa 0,03 bis 1 %, bevorzugt 0,05 bis 0,6 %, besonders bevorzugt 0,06 bis 0,3 %. Erfindungsgemäß von besonderer Bedeutung sind schließlich Inhalationspulver, die etwa 0,08 bis 0,22 % Tiotropium enthalten.

Unter Tiotropium ist das freie Ammoniumkation zu verstehen. Wird im Rahmen der vorliegenden Erfindung der Begriff Wirkstoff verwendet, so ist dies als Bezugnahme auf Tiotropium in Kombination mit einem entsprechenden Gegenion zu verstehen. Als Gegenion (Anion) kommen bevorzugt Chlorid, Bromid, Iodid, Methansulfonat oder para-Toluolsulfonat in Betracht. Von diesen Anionen ist das Bromid bevorzugt. Dementsprechend betrifft die vorliegende Erfindung bevorzugt Inhalationspulver, die zwischen 0,0012 bis 3,6 %, bevorzugt 0,012 bis 2,4 % Tiotropiumbromid enthalten. Erfindungsgemäß von besonderem Interesse sind Inhalationspulver, etwa 0,036 bis 1,2 %, bevorzugt 0,06 bis 0,72 %, besonders bevorzugt 0,072 bis 0,36 % Tiotropiumbromid enthalten. Erfindungsgemäß von besonderer Bedeutung sind Inhalationspulver, die etwa 0,096 bis 0,264 % Tiotropiumbromid enthalten.

Das in den erfindungsgemäßen Inhalationspulvern bevorzugt enthaltene Tiotropiumbromid kann bei der Kristallisation Lösungsmittelmoleküle mit einschließen. Bevorzugt werden zur Herstellung der erfindungsgemäßen Tiotropium-haltigen Inhalationspulver die Hydrate des Tiotropiumbromids eingesetzt. Besonders bevorzugt wird dabei das

aus der WO 02/30928 bekannte kristalline Tiotropiumbromid-monohydrat verwendet. Dieses kristalline Tiotropiumbromid-Monohydrat ist gekennzeichnet durch ein bei der thermischen Analyse mittels DSC auftretendes endothermes Maximum bei 230 ± 5°C bei einer Heizrate von 10K/min. Ferner ist es dadurch gekennzeichnet daß es im IR-Spektrum unter anderem bei den Wellenzahlen 3570, 3410, 3105, 1730, 1260, 1035 und 720 $cm^{-1}$ Banden aufweist. Schließlich weist dieses kristalline Tiotropiumbromidmonohydrat, wie mittels Einkristallröntgenstrukturanalyse bestimmt, eine einfache monoklinische Zelle mit folgenden Dimensionen auf: a =18.0774 Å, b =11.9711 Å, c = 9.9321 Å, β =102.691°, V = 2096.96 $Å^3$.
Dementsprechend betrifft die vorliegende Erfindung bevorzugt Inhaltionspulver, die zwischen 0,0013 bis 3,75 %, bevorzugt 0,0125 bis 2,5 % Tiotropiumbromid-monohydrat enthalten. Erfindungsgemäß von besonderem Interesse sind Inhalationspulver, die etwa 0,0375 bis 1,25 %, bevorzugt 0,0625 bis 0,75 %, besonders bevorzugt 0,075 bis 0,375 % Tiotropiumbromid-monohydrat enthalten. Erfindungsgemäß von besonderer Bedeutung sind schließlich Inhalationspulver, die etwa 0,1 bis 0,275 % Tiotropiumbromidmonohydrat enthalten.

Bei den im Rahmen der vorliegenden Erfindung genannten prozentualen Angaben, handelt es sich stets um Gewichtsprozent, soweit nichts Gegenteiliges spezifisch hervorgehoben wird.

In besonders bevorzugten Inhalationspulvern ist der Hilfsstoff durch eine mittlere Teilchengröße von 12 bis 35 μm, besonders bevorzugt von 13 bis 30 μm gekennzeichnet. Ferner sind insbesondere solche Inhalationspulver besonders bevorzugt, in denen der 10 %-Feinanteil etwa 1 bis 4 μm, bevorzugt etwa 1,5 bis 3 μm beträgt.

Erfindungsgemäß bevorzugt sind ferner solche Inhalationspulver, in denen der Hilfsstoff eine spezifische Oberfläche zwischen 0,2 und 1,5 $m^2/g$, bevorzugt zwischen 0,3 und 1,0 $m^2/g$ besitzt.

Die Hilfsstoffe, die im Sinne der vorliegenden Erfindung zur Anwendung gelangen, werden durch geeignetes Mahlen und/oder Sieben nach gängigen, im Stand der Technik bekannten Verfahren hergestellt. Insbesondere handelt es sich bei den erfindungsgemäß zum Einsatz gelangenden Hilfsstoffen nicht um Hilfsstoffgemische, die durch Mischen von Hilfsstofffraktionen unterschiedlicher mittlerer Teilchengröße erhalten werden.

Als physiologisch unbedenkliche Hilfsstoffe, die zur Darstellung der im Rahmen der erfindungsgemäßen Inhaletten zur Anwendung gelangenden Inhalationspulver Verwendung finden können seien beispielsweise genannt Monosaccharide (z.B. Glucose oder Arabinose), Disaccharide (z.B. Lactose, Saccharose, Maltose, Trehalose), Oligo- und Polysaccharide (z.B. Dextrane), Polyalkohole (z.B. Sorbit, Mannit, Xylit) oder auch Salze (z.B. Natriumchlorid, Calciumcarbonat). Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt als Hilfsstoff Lactose, höchst bevorzugt Lactosemonohydrat zur Anwendung.

Für die erfindungsgemäßen Pulverformulierungen werden bevorzugt Hilfsstoffe hoher Kristallinität verwendet. Diese Kristallinität kann anhand der beim Lösen des Hilfsstoffs freiwerdenden Enthalpie (Lösungsenthalpie) beurteilt. Im Falle des erfindungsgemäß besonders bevorzugt zum Einsatz gelangenden Hilfsstoffs Lactosemonohydrat, wird vorzugsweise Lactose verwendet, die durch eine Lösungsenthalpie von ≥ 45 J/g, bevorzugt von ≥ 50 J/g, besonders bevorzugt von ≥ 52 J/g gekennzeichnet ist.

Die erfindungsgemäßen Inhalationspulver sind entsprechend der der vorliegenden Erfindung zugrunde liegenden Aufgabe gekennzeichnet durch ein hohes Maß an Homogenität im Sinne der Einzeldosierungsgenauigkeit. Diese liegt in einem Bereich von < 8 % , bevorzugt < 6 % , besonders bevorzugt < 4 %.

Nach Einwaage der Ausgangsmaterialien erfolgt die Herstellung der Inhalationspulver aus dem Hilfsstoff und dem Wirkstoff unter Verwendung von im Stand der Technik bekannten Verfahren an. Hierbei sei beispielsweise auf die Offenbarung der WO 02/30390 verwiesen. Die erfindungsgemäßen Inhalationspulver sind dementsprechend beispielsweise gemäß der nachfolgend beschriebenen Vorgehensweise erhältlich. Bei den nachstehend beschriebenen Herstellverfahren werden die genannten Komponenten in den Gewichtsanteilen eingesetzt, wie sie in den zuvor beschriebenen Zusammensetzungen der Inhalationspulver beschrieben wurden.

Zunächst werden der Hilfsstoff und der Wirkstoff in einen geeigneten Mischbehälter eingebracht. Der verwendete Wirkstoff weist eine mittlere Teilchengröße von 0,5 bis 10 μm, vorzugsweise von 1 bis 6 μm, besonders bevorzugt von 2 bis 5 μm auf. Die Zugabe des Wirkstoffs und des Hilfsstoffs erfolgt vorzugsweise über ein Sieb oder einen Siebgranulator mit einer Maschenweite von 0,1 bis 2 mm, besonders bevorzugt 0,3 bis 1 mm, höchst bevorzugt 0,3 bis 0,6 mm. Vorzugsweise wird der Hilfsstoff vorgelegt und anschließend der Wirkstoff in den Mischbehälter eingebracht. Bevorzugt erfolgt bei diesem Mischverfahren die Zugabe der beiden Komponenten portionsweise. Besonders bevorzugt ist das abwechselnde, schichtweise Einsieben der beiden Komponenten. Der Mischvorgang des Hilfsstoffs mit dem Wirkstoff kann bereits während der Zugabe der beiden Komponenten erfolgen. Vorzugsweise wird allerdings erst nach dem schichtweisen Einsieben der beiden Bestandteile gemischt.

Sofern der in das oben beschriebene Verfahren eingesetzte Wirkstoff nicht bereits nach seiner chemischen Herstellung in einer kristallinen Form erhältlich ist, die vorstehend genannte Teilchengrößen aufweist, kann er durch Mahlen in die Teilchengrößen überführt werden, die den vorstehend genannten Parametern genügen (sogenanntes Mikronisieren).

Wird als Wirkstoff das erfindungsgemäß besonders bevorzugte kristalline Tiotropiumbromid-monohydrat eingesetzt, welches durch die WO 02/30928 offenbart wird, hat sich zur Mikronisierung dieser kristallinen Wirkstoffmodifikation insbesondere die nachstehende Vorgehensweise bewährt. Zur Durchführung des Prozesses können gängige Mühlen zum Einsatz gelangen. Bevorzugt wird die Mikronisierung dabei unter Feuchtigkeitsausschluß durchgeführt, besonders bevorzugt unter Einsatz eines entsprechenden Inertgases, wie beispielsweise Stickstoff. Als besonders bevorzugt hat sich die Verwendung von Luftstrahlmühlen erwiesen, in denen die Zerkleinerung des Mahlguts durch Aufeinanderprallen der Partikel miteinender sowie Aufprall der Partikel auf die Wände des Mahlbehälters erfolgt. Als Mahlgas gelangt erfindungsgemäß bevorzugt Stickstoff zur Anwendung. Das Mahlgut wird mittels des Mahlgases unter spezifischen Drücken (Mahldruck) gefördert. Im Rahmen der vorliegenden Erfindung wird der Mahldruck üblicherweise auf einen Wert zwischen etwa 2 und etwa 8 bar, bevorzugt zwischen etwa 3 und etwa 7 bar, besonders bevorzugt zwischen etwa 3,5 und etwa 6,5 bar eingestellt. Der Eintrag des Mahlgutes in die Luftstrahlmühle erfolgt mittels des Speisegases unter spezifischen Drücken (Speisedruck). Im Rahmen der vorliegenden Erfindung hat sich eine Speisedruck zwischen etwa 2 und etwa 8 bar, bevorzugt zwischen etwa 3 und etwa 7 bar, besonders bevorzugt zwischen etwa 3,5 und etwa 6 bar bewährt. Als Speisegas gelangt vorzugsweise ebenfalls ein Inertgas, besonders bevorzugt ebenfalls Stickstoff zur Anwendung. Die Zufuhr des Mahlguts (kristallines Tiotropiumbromidmonohydrat) kann dabei in einer Förderrate von etwa 5 - 35 g/min, vorzugsweise mit etwa 10-30 g/min erfolgen.
Beispielsweise und ohne den Gegenstand der Erfindung darauf zu beschränken, hat sich als eine mögliche Ausführungsform einer Luftstrahlmühle das folgende Gerät bewährt: 2-Zoll Microniser mit Mahlring 0,8 mm-Bohrung, Firma Sturtevant Inc., 348 Circuit Street, Hanover, MA 02239, USA. Unter Verwendung dieses Geräts wird der Mahlprozess vorzugsweise mit folgenden Mahlparametern durchgeführt:
Mahldruck: etwa 4,5 - 6,5 bar; Speisedruck: etwa 4,5 - 6,5 bar: Zufuhr des Mahlguts: etwa 17 - 21 g/min.
Das so erhaltene Mahlgut wird anschließend unter den nachfolgend genannten spezifischen Bedingungen weiterverarbeitet. Hierzu wird das Mikronisat bei einer Temperatur von 15 - 40 °C, vorzugsweise 20 - 35 °C, besonders bevorzugt bei 25 - 30 °C Wasserdampf einer relativen Feuchte von wenigstens 40 % ausgesetzt. Vorzugsweise wird die Feuchte auf einen Wert von 50 - 95 % r.F., bevorzugt auf 60 - 90 % r.F., besonders bevorzugt auf 70 - 80 % r.F. eingestellt.

Unter relativer Feuchte (r.F.) wird hier der Quotient des Partialdruckes des Wasserdampfs und des Dampfdruckes des Wassers bei der betreffenden Temperatur verstanden. Vorzugsweise wird das aus vorstehend beschriebenem Mahlprozess erhältliche Mikronisat den oben genannten Raumbedingungen wenigstens über einen Zeitraum von 6 Stunden ausgesetzt. Bevorzugt wird das Mirkonisat den genannten Raumbedingungen allerdings für etwa 12 bis etwa 48 Stunden, vorzugsweise etwa 18 bis etwa 36 Stunden, besonders bevorzugt etwa 20 bis etwa 28 Stunden ausgesetzt.

Das gemäß vorstehender Vorgehensweise erhältliche erfindungsgemäße Mikronisat des Tiotropiumbromids weist eine mittlere Teilchengröße von zwischen 1,0 μm und 3,5 μm, bevorzugt zwischen 1,1μm und 3,3 μm, besonders bevorzugt zwischen 1,2 μm und 3,0μm und $Q_{(5.8)}$ von größer 60 %, bevorzugt größer 70 %, besonders bevorzugt größer 80 % auf. Dabei bezeichnet der Kennwert $Q_{(5.8)}$ die Teilchenmenge der Partikel, die bezogen auf die Volumenverteilung der Partikel unterhalb von 5.8 μm liegt. Die Partikelgrößen wurden im Rahmen der vorliegenden Erfindung mittels Laserbeugung (Fraunhoferbeugung) bestimmt. Detailliertere Angaben dazu sind den experimentellen Beschreibungen der Erfindung zu entnehmen.

Ebenso charakteristisch für das erfindungsgemäße Tiotropium-Mikronisat, das nach obigem Prozeß dargestellt wurde, sind spezifische Oberflächenwerte im Bereich zwischen 2 $m^2/g$ und 5 $m^2/g$, im besonderen Maße Werte zwischen 2,5 $m^2/g$ und 4,5 $m^2/g$ und in besonders herausragendem Maße zwischen 3,0 $m^2/g$ und 4,0 $m^2/g$.

Ein besonders bevorzugter Aspekt der vorliegenden Erfindung betrifft die erfindungsgemäßen Inhalationspulver, die durch einen Gehalt an vorstehend beschriebenem Tiotropiumbromidmonohydrat-Mikronisat gekennzeichnet sind.

Die vorliegende Erfindung betrifft ferner die Verwendung der erfindungsgemäßen Inhalationspulver zur Herstellung eines Arzneimittels zur Behandlung von Atemwegserkrankungen, insbesondere zur Behandlung von COPD und/oder Asthma.

Die erfindungsgemäßen Inhalationspulver können beispielsweise mittels Inhalatoren appliziert werden, die eine einzelne Dosis aus einem Vorrat mittels einer Meßkammer (z.B. gemäß US 4570630A) oder über andere apparative Vorrichtungen (z.B. gemäß DE 36 25 685 A) dosieren. Vorzugsweise werden die erfindungsgemäßen Inhalationspulver allerdings in Kapseln abgefüllt (zu sogenannten Inhaletten), die in Inhalatoren wie beispielsweise in der WO 94/28958 beschrieben, zur Anwendung gelangen.

Besonders bevorzugt werden die das erfindungsgemäße Inhalationspulver enthaltenden Kapseln mit einem Inhalator appliziert, wie er in Figur 1 dargestellt ist.
Dieser Inhalator ist gekennzeichnet durch ein Gehäuse 1, enthaltend zwei Fenster 2, ein Deck 3, in dem sich Lufteinlaßöffnungen befinden und welches mit einem über ein Siebgehäuse 4 befestigten Sieb 5 versehen ist, eine mit Deck 3 verbundene Inhalationskammer 6, an der ein mit zwei geschliffenen Nadeln 7 versehener, gegen eine Feder 8 beweglicher Drücker 9 vorgesehen ist, ein über eine Achse 10 klappbar mit dem Gehäuse 1, dem Deck 3 und einer Kappe 11 verbundenes Mundstück 12, sowie Luftdurchlaßlöcher 13 zur Einstellung des Strömungswiderstandes.

Die vorliegende Erfindung betrifft ferner die Verwendung der erfindungsgemäßen Inhalationspulver zur Herstellung eines Arzneimittels zur Behandlung von Atemwegserkrankungen, insbesondere zur Behandlung von COPD und/oder Asthma, dadurch gekennzeichnet, daß der vorstehend beschriebene, in Figur 1 dargestellte Inhalator zur Anwendung gelangt.

Für die Anwendung der erfindungsgemäßen Inhalationspulver mittels pulverhaltiger Kapseln werden besonders bevorzugt solche Kapseln verwendet, deren Material ausgewählt ist aus der Gruppe der synthetischen Kunststoffe, besonders bevorzugt ausgewählt aus der Gruppe bestehend aus Polyethylen, Polycarbonat, Polyester, Polypropylen und Polyethylenterephthalat. Besonders bevorzugt sind als synthetische Kunststoffmaterialien Polyethylen, Polycarbonat oder Polyethylenterephthalat. Wird Polyethylen als eines der erfindungsgemäß besonders bevorzugten Kapselmaterialien verwendet, gelangt vorzugsweise Polyethylen mit einer Dichte zwischen 900 und 1000 kg/$m^3$, bevorzugt von 940 - 980 kg/$m^3$, besonders bevorzugt von etwa 960 - 970 kg/$m^3$ (high-density Polyethylen) zur Anwendung.
Die synthetischen Kunststoffe im Sinne der Erfindung können vielseitig mittels dem im Stand der Technik bekannten Herstellverfahren verarbeitet werden. Bevorzugt im Sinne der Erfindung wird die spritzgusstechnische Verarbeitung der Kunststoffe. Besonders bevorzugt wird die Spritzgusstechnik unter Verzicht auf die Verwendung von Formtrennmitteln. Dieses Herstellverfahren ist wohldefiniert und durch eine besonders gute Reproduzierbarkeit gekennzeichnet.

Ein weiterer Aspekt der vorliegenden Erfindung betrifft vorstehend genannte Kapseln, die vorstehend genanntes erfindungsgemäßes Inhalationspulver enthalten. Diese Kapseln können etwa 1 bis 20 mg, bevorzugt etwa 3 bis 15 mg, besonders bevorzugt etwa 4 bis 12 mg Inhalationspulver enthalten. Erfindungsgemäß bevorzugte Formulierungen enthalten 4 bis 6 mg Inhalationspulver. Von erfindungsgemäß gleichrangiger Bedeutung sind Inhaltionskapseln, die die die erfindungsgemäßen Formulierungen in einer Menge von 8 bis 12 mg enthalten.

Ferner betrifft die vorliegende Erfindung ein Inhalationskit, bestehend aus einer oder mehrerer der vorstehend beschriebenen, durch einen Gehalt an erfindungsgemäßem Inhalationspulver gekennzeichneten Kapseln in Verbindung mit dem Inhalator gemäß Figur 1.

Die vorliegende Erfindung betrifft ferner die Verwendung der vorstehend genannten, durch einen Gehalt an erfindungsgemäßem Inhalationspulver gekennzeichneten Kapseln, zur Herstellung eines Arzneimittels zur Behandlung von Atemwegserkrankungen, insbesondere zur Behandlung von COPD und/oder Asthma.

Die Darstellung von befüllten Kapseln, die die erfindungsgemäßen Inhalationspulver enthalten, erfolgt nach im Stand der Technik bekannten Verfahren durch Befüllung der leeren Kapseln mit den erfindungsgemäßen Inhalationspulvern.

Die folgenden Beispiele dienen einer weitergehenden Erläuterung der vorliegenden Erfindung, ohne den Umfang der Erfindung allerdings auf die nachfolgenden beispielhaften Ausführungsformen zu beschränken.

## Ausgangsmaterialien

### I) Hilfsstoff:

In den nachfolgenden Beispielen wird als Hilfsstoff Lactose-Monohydrat verwendet. Dieser kann beispielsweise von der Firma Borculo Domo Ingredients, Borculo/NL unter der Produktbezeichnung *Lactochem Extra Fine Powder* bezogen werden. Die erfindungsgemäßen Spezifikationen für die Teilchengröße und die spezifische Oberfläche werden von dieser Lactosequalität erfüllt. Ferner weist diese Lactose die vorstehend genannten, für Lactose erfindungsgemäß bevorzugten Lösungsenthalpie-Werte auf.

### II) Mikronisierung von kristallinem Tiotropiumbromid-monohydrat:

Das gemäß der WO 02/30928 erhältliche kristalline Tiotropiumbromid-monohydrat wird mit einer Luftstrahlmühle vom Typ 2-Zoll Microniser mit Mahlring 0,8 mm-Bohrung, Firma Sturtevant Inc., 348 Circuit Street, Hanover, MA 02239, USA mikronisiert. Unter Verwendung von Stickstoff als Mahlgas werden dabei beispielsweise die folgenden Mahlparameter eingestellt:
Mahldruck: 5,5 bar; Speisedruck: 5,5 bar;
Zufuhr (des kristallinen Monohydrats) bzw. Fließgeschwindigkeit: 19 g/min.

Das erhaltene Mahlgut wird anschließend auf Hordenblechen in einer Schichtdicke von etwa 1 cm ausgebreitet und für 24 - 24,5 Stunden den folgenden Klimabedingungen unterworfen:
Temperatur: 25 - 30 °C; Relative Feuchte: 70-80 %.

**Meßmethoden:**

**I) Partikelgrößenbestimmung von Tiotropium Monohydrat, mikronisiert:**

Messgerät und Einstellungen:

Die Bedienung der Geräte erfolgte in Übereinstimmung mit den Bedienungsanleitungen des Herstellers.

| | |
|---|---|
| Messgerät: | Laser-Beugungs-Spektrometer (HELOS), Sympatec |
| Dispergiereinheit: | Trockendispergierer RODOS mit Saugtrichter,Sympatec |
| Probenmenge: | 200 mg ± 150 mg |
| Produktzufuhr: | Schwingrinne Vibri, Fa. Sympatec |
| Frequenz d. Vibrationsrinne: | bis 100 % ansteigend |
| Dauer der Probenzufuhr: | 15 bis 25 sek. (im Fall von 200 mg) |
| Brennweite: | 100 mm (Messbereich: 0,9 - 175 μm) |
| Messzeit/Wartezeit: | ca. 15 s (im Fall von 200 mg) |
| Zykluszeit: | 20 ms |
| Start/Stop bei: | 1 % auf Kanal 28 |
| Dispergiergas: | Druckluft |
| Druck: | 3 bar |
| Unterdruck: | maximal |
| Auswertemodus: | HRLD |

Probenvorbereitung / Produktzufuhr:

Ca. 200 mg der Prüfsubstanz werden auf einem Kartenblatt eingewogen.
Mit einem weiteren Kartenblatt werden alle größeren Agglomerate zerstoßen.
Das Pulver wird dann auf der vorderen Hälfte der Schwingrinne (ab ca. 1 cm vom vorderen Rand) fein verteilt aufgestreut. Nach dem Start der Messung wird die Frequenz der Schwingrinne so variiert, dass die Zufuhr der Probe möglichst kontinuierlich erfolgt. Die Produktmenge darf aber auch nicht zu groß sein damit eine ausreichende Dispergierung erreicht wird.

**II) Partikelgrößenbestimmung der Laktose:**

Messgerät und Einstellungen

Die Bedienung der Geräte erfolgte in Übereinstimmung mit den Bedienungsanleitungen des Herstellers.

| | |
|---|---|
| Messgerät: | Laser-Beugungs-Spektrometer (HELOS), Sympatec |
| Dispergiereinheit: | Trockendispergierer RODOS mit Saugtrichter, Sympatec |
| Probenmenge: | 200 mg ± 100 mg |
| Produktzufuhr: | Vibrationsrinne Typ VIBRI, Sympatec |
| Frequenz d. Vibrationsrinne: | 100 % ansteigend |
| Brennweite: | 200 mm (Messbereich: 1,8 - 350 μm) |
| Messzeit/Wartezeit: | ca. 10 s (im Falle von 200 mg) |
| Zykluszeit: | 10 ms |
| Start/Stop bei: | 1 % auf Kanal 28 |
| Dispergiergas: | Druckluft |
| Druck: | 3 bar |
| Unterdruck: | maximal |
| Auswertemodus: | HRLD |

Probenvorbereitung / Produktzufuhr:

Ca. 200 mg der Prüfsubstanz werden auf einem Kartenblatt eingewogen. Mit einem weiteren Kartenblatt werden alle größeren Agglomerate zerstoßen. Das Pulver wird in die Vibrationsrinne überführt. Es wird ein Abstand von 1.2 bis 1.4 mm zwischen Vibrationsrinne und Trichter eingestellt. Nach dem Start der Messung wird die Amplitudeneinstellung der Schwingrinne möglichst kontinuierlich auf 100 % gegen Ende der Messung gesteigert.

**III) Bestimmung der spezifischen Oberfläche von Tiotropiumbromid-Monohydrat, mikronisiert (1-Punkt-BET-Methode):**

Prinzip

Die Bestimmung der spezifischen Oberfläche erfolgt, indem die Pulverprobe einer Stickstoff/Heliumatmosphäre bei unterschiedlichen Drücken ausgesetzt wird. Durch Abkühlung der Probe erfolgt eine Kondensation der Stickstoffmolekülen auf der Oberfläche der Partikel. Die kondensierte Stickstoffmenge wird über die Änderung der thermischen Leitfähigkeit des Stickstoff/Heliumgemisches bestimmt und die Oberfläche der Probe über den Flächenbedarf von Stickstoff bestimmt. Über diesen Wert und die Probeneinwaage wird die spezifische Oberfläche berechnet.

Messgerät und Einstellungen

| | |
|---|---|
| Messgerät: | Monosorb, Fa. Quantachrome |
| Ausheizgerät: | Monotektor, Fa. Quantachrome |
| Mess- und Trockengas: | Stickstoff (5.0) / Helium (4.6) 70/30, Fa. Messer Griesheim |
| Adsorbat: | Stickstoff 30 %-ig in Helium |
| Kältemittel: | flüssiger Stickstoff |
| Messzelle: | mit Kapillarrohr, Fa. W. Pabisch GmbH&Co.KG |
| Kalibrierspritze; | 1000 µl, Fa. Precision Sampling Corp. |
| Analysenwaage: | R 160 P, Fa. Satorius |

Berechnung der spezifischen Oberfläche:

Die Messwerte werden vom Gerät in [$m^2$] angezeigt und werden i.d.R. in [$cm^2/g$] auf die Einwaage (Trockenmasse) umgerechnet:

$$A_{spez} = \frac{MW * 10000}{m_{tr}}$$

$A_{spez}$ = spezifische Oberfläche [$cm^2/g$]
MW = Messwert [$m^2$]
$m_{tr}$ = Trockenmasse [g]
10000 = Umrechnungsfaktor [$cm^2/m^2$]

**IV) Bestimmung der spezifischen Oberfläche der Laktose (Mehrpunkt-BET-Methode):**

Prinzip

Die Bestimmung der spezifischen Oberfläche erfolgt, indem die Pulverprobe einer Stickstoffatmosphäre bei unterschiedlichen Drücken ausgesetzt wird. Durch Abkühlung der Probe erfolgt eine Kondensation der Stickstoffmoleküle auf der Oberfläche der Partikel. Die kondensierte Stickstoffmenge wird über den Druckabfall im System bestimmt und die spezifische Oberfläche der Probe über den Flächenbedarf von Stickstoff und der Probeneinwaage berechnet.

Die Bedienung der Geräte erfolgte in Übereinstimmung mit den Bedienungsanleitungen des Herstellers.

Meßgerät und Einstellungen

| | |
|---|---|
| Meßgerät | Tri Star Multi Point BET, Fa. Micromeritics |
| Ausheizstation: | VacPrep 061, Fa. Micromeritics |
| Ausheizen: | ca. 12h / 40°C |
| Sample tube: | ½ inch; use filler rod |
| Analysis Condition: | 10 point BET surface 0,1 to 0,20 p/p0 |
| Absolute P. tolerance: | 5,0 mmHg |
| rel. P. tolerance: | 5,0 % |
| Evacuation rate: | 50,0 mmHg/sec. |
| Unvestticted evac f.: | 10,0 mmHg |
| Evac. time: | 0,1 hours |
| Free Space: | Lower Dewar, time: 0,5 h |
| Equilibration interv.: | 20 sec |
| Min. equl. delay: | 600 sec |
| Adsorptive: | Nitrogen |

**V) Bestimmung der Lösungswärme der Laktose (Lösungsenthalpie) $E_c$:**

Die Bestimmung der Lösungsenthalpie erfolgt mittels eines Lösungskalorimeter 2225 *Precision Solution Calorimeter* der Fa. Thermometric .
Die Lösungswärme wird anhand der - infolge des Löseprozesses - auftretende Temperaturänderung und der aus der Basislinie berechneten systembedingten Temperaturänderung berechnet. Vor und nach dem Ampullenbruch wird jeweils eine elektrische Kalibrierung mit einem integrierten Heizwiderstand genau bekannter Leistung durchgeführt. Hierbei wird eine bekannte Wärmeleistung über einen festgelegten Zeitraum an das System abgegeben und der Temperatursprung ermittelt.

Messgerät und Einstellungen

| | |
|---|---|
| Lösungskalorimeter: | 2225 Precision Solution Calorimeter, Fa. Thermometric |
| Reaktionszelle: | 100 ml |
| Thermistorwiderstand: | 30,0 kΩ (bei 25 °C) |
| Rührergeschwindigkeit: | 500 U/min |
| Thermostat: | Thermostat des 2277 Thermal Activity Monitor TAM, Fa. Thermometric |
| Temperatur: | 25 °C ± 0.0001 °C (über 24h) |
| Meßampullen: | Crushing ampoules 1 ml, Fa. Thermometric |
| Dichtung: | Silikonstopfen und Bienenwachs, Fa. Thermometric |
| Einwaage: | 40 bis 50 mg |
| Lösemittel: | Wasser, chemisch rein |
| Volumen Lösemittel: | 100 ml |
| Badtemperatur: | 25°C |
| Temperaturauflösung: | High |
| Starttemperatur: | -40mm (± 10mK) temperature-offset |
| Interface: | 2280-002 TAM accessory interface 50 Hz, Fa. Thermometric |
| Software: | SolCal V 1.1 für WINDOWS |

(fortgesetzt)

| | |
|---|---|
| Auswertung: | Automatische Auswertung mit Menüpunkt CALCULATION/ ANALYSE EXPERIMENT. (Dynamik der Basislinie ; Kalibrierung nach dem Ampullenbruch). |

Elektrische Kalibrierung:

Die elektrische Kalibrierung erfolgt während der Messung, einmal vor und einmal nach dem Ampullenbruch. Zur Auswertung wird die Kalibrierung nach dem Ampullenbruch herangezogen.

| | |
|---|---|
| Wärmemenge: | 2,5 J |
| Heizleistung: | 500 mW |
| Heizdauer: | 10 s |
| Dauer der Basislinien: | 5 min (vor und nach Heizen) |

**Darstellung der erfindungsgemäßen Pulverformulierungen:**

I) Apparatives

Zur Herstellung der Inhalationspulver können beispielsweise die folgenden Maschinen und Geräte Verwendung finden:

Mischbehälter bzw. Pulvermischer: Turbulamischer 2 L, Typ 2C; Hersteller Willy A. Bachofen AG, CH-4500 Basel

Handsieb: 0,135 mm Maschenweite

Die Befüllung der leeren Inhaltionskapseln mittels Tiotropium-haltigen Inhaltionspulver kann händisch oder maschinell erfolgen. Es können nachstehende Geräte verwendet werden.

Kapselfüllmaschine:

MG2, Typ G100, Hersteller: MG2 S.r.l, I-40065 Pian di Macina di Pianoro (BO), Italien

**Beispiel 1**:

Pulvermischung :

Zur Herstellung der Pulvermischung werden 299,39 g Hilfsstoff und 0,61 g mikronisiertes Tiotropiumbromid-monohydrat eingesetzt. In den daraus erhaltenen 300 g Inhalationspulver beträgt der Wirkstoffanteil 0,2 % (bezogen auf Tiotropium).

Über ein Handsieb mit einer Maschenweite von 0,315 mm werden in einen geeigneten Mischbehälter ca. 40-45 g Hilfsstoff vorgelegt. Anschließend werden abwechselnd Tiotropiumbromid-monohydrat in Portionen von ca. 90-110 mg und Hilfsstoff in Portionen von etwa 40-45 g schichtweise eingesiebt. Die Zugabe des Hilfsstoffs und des Wirkstoffs erfolgt in 7 bzw. 6 Schichten.

Die eingesiebten Bestandteile werden anschließend gemischt (Mischen: 900 Umdrehungen). Die Endmischung wird noch zweimal über ein Handsieb gegeben und anschließend jeweils gemischt (Mischen: 900 Umdrehungen).

Gemäß der in Beispiel 1 beschriebenen Vorgehensweise können solche Inhalationspulver erhalten werden, die nach Befüllung der entsprechenden Kunststoffkapseln beispielsweise zu den nachstehenden Inhalationskapseln führen:

**Beispiel 2:**

| | |
|---|---|
| Tiotropiumbromid-monohydrat: | 0,0113 mg |

(fortgesetzt)

| | |
|---|---|
| Lactose Monohydrat*): | 5,4887 mg |
| Polyethylen-Kapseln: | 100,0 mg |
| Total: | 105,5 mg |

*) der Hilfsstoff ist durch die folgenden Parameter **gekennzeichnet:**
mittlere Teilchengröße: 17,9 µm;
10 %-Feinanteil: 2,3 µm;
spezifische Oberfläche: 0,61 $m^2/g$;

**Beispiel 3:**

| | |
|---|---|
| Tiotropiumbromid-monohydrat: | 0,0113 mg |
| Lactose Monohydrat*): | 5,4887 mg |
| Polyethylen-Kapseln: | 100,0 mg |
| Total: | 105,5 mg |

*) der Hilfsstoff ist durch die folgenden Parameter **gekennzeichnet:**
mittlere Teilchengröße: 18,5 µm;
10 %-Feinanteil: 2,2 µm;
spezifische Oberfläche: 0,83 $m^2/g$;

**Beispiel 4:**

| | |
|---|---|
| Tiotropiumbromid-monohydrat: | 0,0113 mg |
| Lactose Monohydrat*): | 5,4887 mg |
| Polyethylen-Kapseln: | 100,0 mg |
| Total: | 105,5 mg |

*) der Hilfsstoff ist durch die folgenden Parameter **gekennzeichnet:**
mittlere Teilchengröße: 21,6 µm;
10 %-Feinanteil: 2,5 µm;
spezifische Oberfläche: 0,59 $m^2/g$;

**Beispiel 5:**

| | |
|---|---|
| Tiotropiumbromid-monohydrat: | 0,0113 mg |
| Lactose Monohydrat*): | 5,4887 mg |
| Polyethylen-Kapseln: | 100,0 mg |
| Total: | 105,5 mg |

*) der Hilfsstoff ist durch die folgenden Parameter **gekennzeichnet:**
mittlere Teilchengröße: 16,0 µm;
10 %-Feinanteil: 2,0 µm;
spezifische Oberfläche: 0,79 $m^2/g$;

**Beispiel 6:**

| | |
|---|---|
| Tiotropiumbromid-monohydrat: | 0,0225 mg |
| Lactose Monohydrat*): | 5,4775 mg |
| Polyethylen-Kapseln: | 100,0 mg |
| Total: | 105,5 mg |

*) der Hilfsstoff ist durch die folgenden Parameter **gekennzeichnet:**

| | |
|---|---|
| mittlere Teilchengröße: | 17,9 µm; |
| 10 %-Feinanteil: | 2,3 µm; |
| spezifische Oberfläche: | 0,61 $m^2$/g; |

**Beispiel 7:**

| | |
|---|---|
| Tiotropiumbromid-monohydrat: | 0,0225 mg |
| Lactose Monohydrat*): | 5,4775 mg |
| Polyethylen-Kapseln: | 100,0 mg |
| Total: | 105,5 mg |

*) der Hilfsstoff ist durch die folgenden Parameter **gekennzeichnet:**

| | |
|---|---|
| mittlere Teilchengröße: | 18,5 µm; |
| 10 %-Feinanteil: | 2,2 µm; |
| spezifische Oberfläche: | 0,83 $m^2$/g; |

**Beispiel 8:**

| | |
|---|---|
| Tiotropiumbromid-monohydrat: | 0,0225 mg |
| Lactose Monohydrat*): | 5,4775 mg |
| Polyethylen-Kapseln: | 100,0 mg |
| Total: | 105,5 mg |

*) der Hilfsstoff ist durch die folgenden Parameter **gekennzeichnet:**

| | |
|---|---|
| mittlere Teilchengröße: | 21,6 µm; |
| 10 %-Feinanteil: | 2,5 µm; |
| spezifische Oberfläche: | 0,59 $m^2$/g; |

**Beispiel 9:**

| | |
|---|---|
| Tiotropiumbromid-monohydrat: | 0,0225 mg |
| Lactose Monohydrat*): | 5,4775 mg |
| Polyethylen-Kapseln: | 100,0 mg |
| Total: | 105,5 mg |
| *) der Hilfsstoff ist durch die folgenden Parameter **gekennzeichnet:** | |

| | |
|---|---|
| mittlere Teilchengröße: | 16,0 μm; |
| 10 %-Feinanteil: | 2,0 μm; |
| spezifische Oberfläche: | 0,79 $m^2/g$; |

**Beispiel 10:**

| | |
|---|---|
| Tiotropiumbromid-monohydrat: | 0,0056 mg |
| Lactose Monohydrat*): | 5,4944 mg |
| Polyethylen-Kapseln: | 100,0 mg |
| Total: | 105,5 mg |
| *) der Hilfsstoff ist durch die folgenden Parameter **gekennzeichnet:** | |

| | |
|---|---|
| mittlere Teilchengröße: | 17,9 μm; |
| 10 %-Feinanteil: | 2,3 μm; |
| spezifische Oberfläche: | 0,61 $m^2/g$; |

**Beispiel 11:**

| | |
|---|---|
| Tiotropiumbromid-monohydrat: | 0,0056 mg |
| Lactose Monohydrat*): | 5,4944 mg |
| Polyethylen-Kapseln: | 100,0 mg |
| Total: | 105,5 mg |
| *) der Hilfsstoff ist durch die folgenden Parameter **gekennzeichnet:** | |

| | |
|---|---|
| mittlere Teilchengröße: | 18,5 μm; |
| 10 %-Feinanteil: | 2,2 μm; |
| spezifische Oberfläche: | 0,83 $m^2/g$; |

**Beispiel 12:**

| | |
|---|---|
| Tiotropiumbromid-monohydrat: | 0,0056 mg |
| Lactose Monohydrat*): | 5,4944 mg |

(fortgesetzt)

| | |
|---|---|
| Polyethylen-Kapseln: | 100,0 mg |
| Total: | 105,5 mg |

*) der Hilfsstoff ist durch die folgenden Parameter **gekennzeichnet:**

| | |
|---|---|
| mittlere Teilchengröße: | 21,6 μm; |
| 10 %-Feinanteil: | 2,5 μm; |
| spezifische Oberfläche: | 0,59 $m^2/g$; |

**Beispiel 13:**

| | |
|---|---|
| Tiotropiumbromid-monohydrat: | 0,0056 mg |
| Lactose Monohydrat*): | 5,4944 mg |
| Polyethylen-Kapseln: | 100,0 mg |
| Total: | 105,5 mg |

*) der Hilfsstoff ist durch die folgenden Parameter **gekennzeichnet:**

| | |
|---|---|
| mittlere Teilchengröße: | 16,0 μm; |
| 10 %-Feinanteil: | 2,0 μm; |
| spezifische Oberfläche: | 0,79 $m^2/g$; |

**Beispiel 14:**

| | |
|---|---|
| Tiotropiumbromid-monohydrat: | 0,0056 mg |
| Lactose Monohydrat*): | 9,9944 mg |
| Polyethylen-Kapseln: | 100,0 mg |
| Total: | 110,0 mg |

*) der Hilfsstoff ist durch die folgenden Parameter **gekennzeichnet:**

| | |
|---|---|
| mittlere Teilchengröße: | 17,9 μm; |
| 10 %-Feinanteil: | 2,3 μm; |
| spezifische Oberfläche: | 0,61 $m^2/g$; |

**Beispiel 15:**

| | |
|---|---|
| Tiotropiumbromid-monohydrat: | 0,0113 mg |
| Lactose Monohydrat*): | 9,9887 mg |
| Polyethylen-Kapseln: | 100,0 mg |

(fortgesetzt)

| | |
|---|---|
| Total: | 110,0 mg |
| *) der Hilfsstoff ist durch die folgenden Parameter **gekennzeichnet:** | |

| | |
|---|---|
| mittlere Teilchengröße: | 18,5 μm; |
| 10 %-Feinanteil: | 2,2 μm; |
| spezifische Oberfläche: | 0,83 $m^2$/g; |

**Beispiel 16:**

| | |
|---|---|
| Tiotropiumbromid-monohydrat: | 0,0225 mg |
| Lactose Monohydrat*): | 9,9775 mg |
| Polyethylen-Kapseln: | 100,0 mg |
| Total: | 110,0 mg |
| *) der Hilfsstoff ist durch die folgenden Parameter gekennzeichnet: | |

| | |
|---|---|
| mittlere Teilchengröße: | 21,6 μm; |
| 10 %-Feinanteil: | 2,5 μm; |
| spezifische Oberfläche: | 0,59 $m^2$/g; |

**Beispiel 17:**

| | |
|---|---|
| Tiotropiumbromid-monohydrat: | 0,0125 mg |
| Lactose Monohydrat*): | 9,9875 mg |
| Polyethylen-Kapseln: | 100,0 mg |
| Total: | 110,0 mg |
| *) der Hilfsstoff ist durch die folgenden Parameter gekennzeichnet: | |

| | |
|---|---|
| mittlere Teilchengröße: | 17,9 μm; |
| 10 %-Feinanteil: | 2,3 μm; |
| spezifische Oberfläche: | 0,61 $m^2$/g; |

**Bespiel 18:**

| | |
|---|---|
| Tiotropiumbromid-monohydrat: | 0,0125 mg |
| Lactose Monohydrat*): | 9,9875 mg |
| Polyethylen-Kapseln: | 100,0 mg |

(fortgesetzt)

| | |
|---|---|
| Total: | 110,0 mg |
| *) der Hilfsstoff ist durch die folgenden Parameter gekennzeichnet: | |

| | |
|---|---|
| mittlere Teilchengröße: | 18,5 μm; |
| 10 %-Feinanteil: | 2,2 μm; |
| spezifische Oberfläche: | 0,83 $m^2/g$; |

**Beispiel 19:**

| | |
|---|---|
| Tiotropiumbromid-monohydrat: | 0,0125 mg |
| Lactose Monohydrat*): | 9,9875 mg |
| Polyethylen-Kapseln: | 100,0 mg |
| Total: | 110,0 mg |
| *) der Hilfsstoff ist durch die folgenden Parameter **gekennzeichnet:** | |

| | |
|---|---|
| mittlere Teilchengröße: | 21,6 μm; |
| 10 %-Feinanteil: | 2,5 μm; |
| spezifische Oberfläche: | 0,59 $m^2/g$; |

**Beispiel 20**:

| | |
|---|---|
| Tiotropiumbromid-monohydrat: | 0,0125 mg |
| Lactose Monohydrat*): | 9,9875 mg |
| Polyethylen-Kapseln: | 100,0 mg |
| Total: | 110,0 mg |
| *) der Hilfsstoff ist durch die folgenden Parameter **gekennzeichnet:** | |

| | |
|---|---|
| mittlere Teilchengröße: | 16,0 μm; |
| 10 %-Feinanteil: | 2,0 μm; |
| spezifische Oberfläche: | 0,79 $m^2/g$; |

## Patentansprüche

1. Abgepackte Inhalationspulver enthaltend 0,001 bis 3 % Tiotropium im Gemisch mit einem physiologisch unbedenklichen Hilfsstoff ausgewählt ist aus der Gruppe der Monosaccharide, der Disaccharide, der Oligo- und Polysaccharide, der Polyalkohole oder auch der Salze, **dadurch gekennzeichnet, dass** der Hilfsstoff eine mittlere Teilchengröße von 10 - 50 μm, einen 10 %-Feinanteil von 0,5 bis 6 μm sowie eine spezifischen Oberfläche von 0,1 bis 2 $m^2/g$ aufweist, wobei eine Einzeldosis in ein Pulverreservoir abgefüllt ist, und die Pulverreservoire aus einem Material aus synthetischem Kunststoff bestehen.

2. Inhalationspulver nach Anspruch 1, **dadurch gekennzeichnet, dass** das Tiotropium in Form seines Chlorids, Bromids, Iodids, Methansulfonats oder para-Toluolsulfonats enthalten ist.

3. Inhalationspulver nach Anspruch 2, **dadurch gekennzeichnet, dass** der physiologisch unbedenkliche Hilfsstoff ausgewählt ist aus der Gruppe bestehend aus Glucose, Arabinose, Lactose, Saccharose, Maltose und Trehalose, gegebenenfalls in Form ihrer Hydrate.

4. Kapsel enthaltend ein Inhalationspulver nach einem der Ansprüche 1 bis 3.

5. Kapsel nach Anspruch 4, **dadurch gekennzeichnet, dass** das Kapselmaterial aus synthetischem Kunststoff besteht.

6. Kapsel nach Anspruch 5, **dadurch gekennzeichnet, dass** das Kapselmaterial ausgewählt ist aus der Gruppe bestehend aus Polyethylen, Polycarbonat, Polyester, Polypropylen und Polyethylenterephthalat.

7. Kapsel nach einem der Ansprüche 4 bis 6, **dadurch gekennzeichnet, dass** sie etwa 1 bis 20 mg des Inhalationspulvers nach einem der Ansprüche 1 bis 3 enthalten.

8. Verwendung eines Inhalationspulvers gemäß einem der Ansprüche 1 bis 3 zur Herstellung eines Arzneimittels zur Behandlung von Atemwegserkrankungen, insbesondere zur Behandlung von COPD und/oder Asthma.

9. Inhalationskit bestehend aus einer Kapsel nach einem der Ansprüche 4 bis 7 und einem Inhalator, der zur Applikation von Inhalationspulvern aus pulverhaltigen Kapseln verwendet werden kann.

## Claims

1. Packaged inhalable powder containing 0.001 to 3% of tiotropium in admixture with a physiologically acceptable excipient selected from among the monosaccharides, disaccharides, oligo- and polysaccharides, polyalcohols or salts, **characterised in that** the excipient has an average particle size of 10 - 50 µm, a 10 % fine content of 0.5 to 6 µm and a specific surface of 0.1 to 2 $m^2/g$, wherein a single dose has been packed into a powder reservoir and the powder reservoirs are composed of a material made from synthetic plastics.

2. Inhalable powder according to claim 1, **characterised in that** the tiotropium is present in the form of the chloride, bromide, iodide, methanesulphonate or para-toluenesulphonate thereof.

3. Inhalable powder according to claim 2, **characterised in that** the physiologically acceptable excipient is selected from among glucose, arabinose, lactose, saccharose, maltose and trehalose, optionally in the form of the hydrates thereof.

4. Capsule containing an inhalable powder according to one of claims 1 to 3.

5. Capsule according to claim 4, **characterised in that** the capsule material consists of synthetic plastics.

6. Capsule according to claim 5, **characterised in that** the capsule material is selected from among polyethylene, polycarbonate, polyester, polypropylene and polyethylene terephthalate.

7. Capsule according to one of claims 4 to 6, **characterised in that** it contains about 1 to 20 mg of the powder for inhalation according to one of claims 1 to 3.

8. Use of an inhalable powder according to one of claims 1 to 3 for preparing a medicament for the treatment of respiratory complaints, particularly for treating COPD and/or asthma.

9. Inhalation kit consisting of a capsule according to one of claims 4 to 7 and an inhaler which can be used for administering inhalable powders from powder-filled capsules.

## Revendications

**1.** Poudres pour inhalation emballées contenant de 0,001 à 3 % de tiotropium en mélange avec un adjuvant physiologiquement inoffensive choisi parmi le groupe des monosaccharides, des disaccharides, des oligo- et des polysaccharides, des polyalcools ou encore des sels, **caractérisées en ce que** l'adjuvant présente une dimension moyenne des particules de 10 à 50 μm, une part de fines de 0,5 à 10 μm de 10 % ainsi qu'une surface spécifique de 0,1 à 2 $m^2/g$, une dose individuelle étant remplie dans un réservoir de poudre et les réservoirs de poudre se composant d'un matériau en matière plastique synthétique.

**2.** Poudres pour inhalation selon la revendication 1, **caractérisées en ce que** le tiotropium est contenu sous la forme de son chlorure, de son bromure, de son iodure, de son méthane sulfonate ou de son paratoluène sulfonate.

**3.** Poudres pour inhalation selon la revendication 2, **caractérisées en ce que** l'adjuvant physiologiquement inoffensive est choisi parmi le groupe consistant de glucose, d'arabinose, de lactose, de saccharose, de maltose et de tréhalose, le cas échéant sous la forme de leurs hydrates.

**4.** Capsule contenant une poudre pour inhalation selon l'une quelconque des revendications 1 à 3.

**5.** Capsule selon la revendication 4, **caractérisée en ce que** le matériau de la capsule consiste en plastique synthétique.

**6.** Capsule selon la revendication 5, **caractérisée en ce que** le matériau de la capsule est choisi parmi le groupe se composant de polyéthylène, de polycarbonate, de polyester, de polypropylène et de poly(téréphtalate d'éthylène).

**7.** Capsule selon l'une quelconque des revendications 4 à 6, **caractérisée en ce qu'**elle contient environ 1 à 20 mg de la poudre pour inhalation selon l'une quelconque des revendications 1 à 3.

**8.** Utilisation d'une poudre pour inhalation selon l'une quelconque des revendications 1 à 3 pour la préparation d'un médicament pour le traitement des affections des voies respiratoires, en particulier pour le traitement du BPCO et/ou de l'asthme.

**9.** Kit d'inhalation se composant d'une capsule selon l'une quelconque des revendications 4 à 7, et d'un inhalateur qui peut être utilisé pour administrer des poudres pour inhalation provenant de capsules contenant de la poudre.

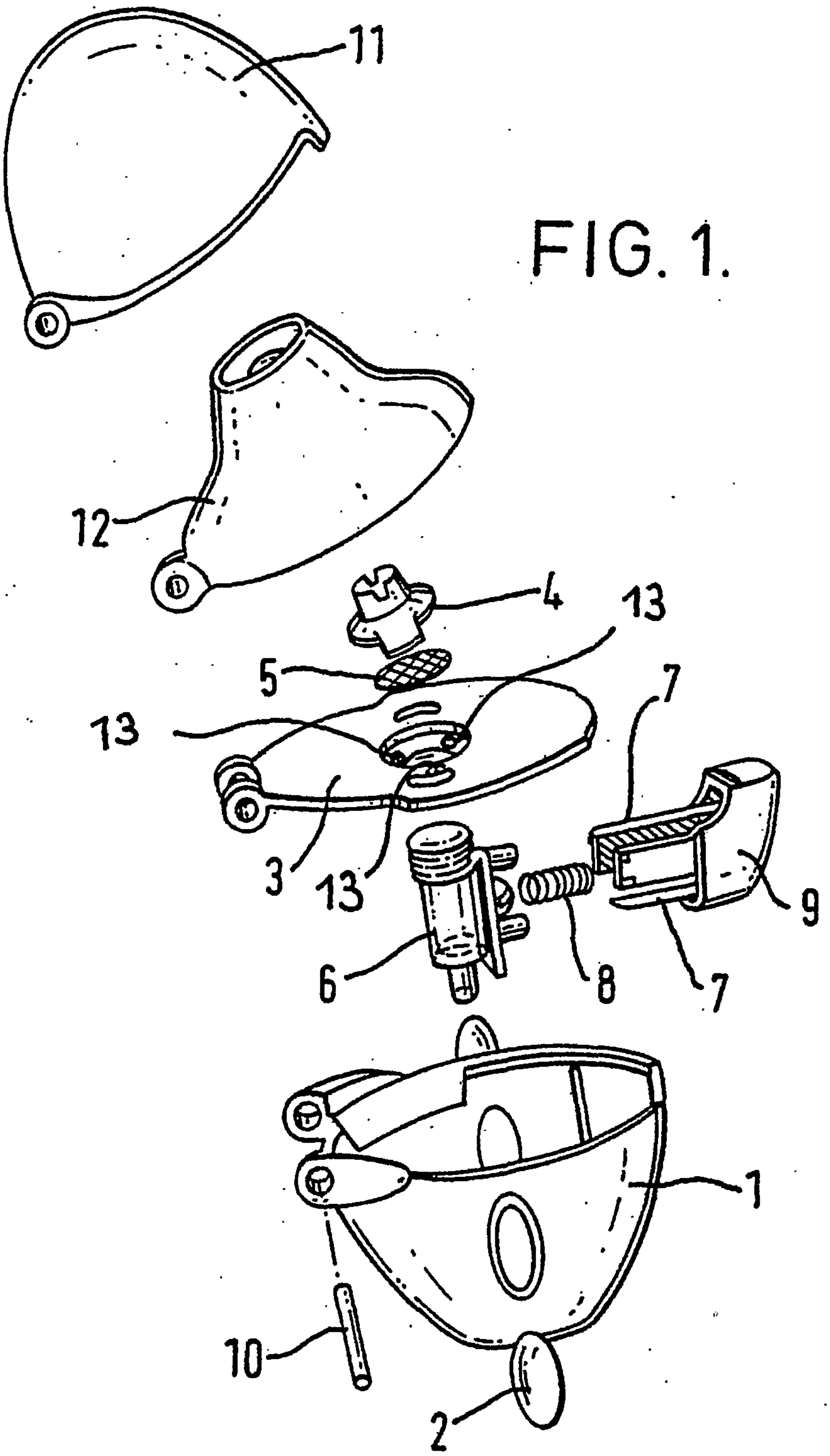


FIG. 1.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 418716 A1
- WO 9428958 A
- WO 0230389 A
- WO 0230928 A
- WO 0230390 A
- US 4570630 A
- DE 3625685 A